# EUROPEAN PATENT APPLICATION

(11) **EP 3 967 311 A1**
(43) Date of publication of application: **16.03.2022**
(21) Application number: 20195704.0
(22) Date of filing: 11.09.2020
(51) Int. Cl.: A61K 31/497, A61K 31/4178, A61K 45/06, A61P 43/00

(54) **COMPOUNDS FOR USE IN THE TREATMENT OF DRY MOUTH**

(71) Applicant: Astellas Pharma Inc., Chuo-ku Tokyo 103-8411 (JP)
(72) Inventor: de Vries, Michiel Henricus, 1381EC Weesp (NL); Groenendaal, Dorien, 2134 AT Hoofddorp (NL)
(74) Representative: Hoffmann Eitle

(57) **Abstract**

The present invention is concerned with the use of compounds with Formula (I) in the treatment of dry mouth.

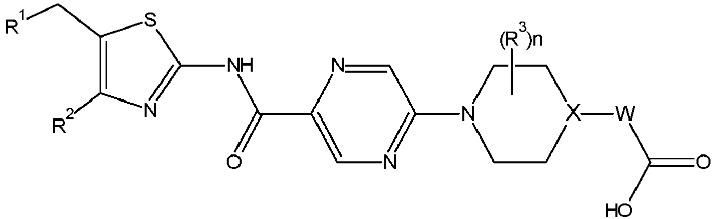

## Description

The present invention relates to compounds for use in the treatment of dry mouth.

Dry mouth and oral dryness are general terms that encompass two medical entities: xerostomia and hyposalivation. Xerostomia is the subjective complaint of oral dryness and is medically classified as a symptom. Hyposalivation is the objective reduction in salivary secretion, as the consequence of reduced salivary gland function. Generally, these conditions are closely related to each other: a reduced salivary flow is the most frequent cause of xerostomia, and it is generally accepted that hyposalivation causes the symptom of oral dryness when salivary secretion rate is reduced by at least 50% (https://www.eaom.eu/pdf/content/dry mouth.pdf). Dry mouth can also cause other symptoms, such as rough tongue, mouth sores, and cracked lips. It may also lead to difficulties in swallowing.

Dry mouth can have many causes. It often results from dehydration. Radiation therapy for head and neck cancer often damages salivary glands and causes hyposalivation. Some diseases, such as diabetes and Sjogren syndrome, can also affect saliva production. More seriously, dry mouth can be a side effect of certain medications. In fact, there are more than 1,800 commonly prescribed medications that list dry mouth as a side effect.

Dry mouth is especially noteworthy as a side effect of anticholinergic drugs. Salivary secretion is under the control of autonomic nervous activity, the rate of fluid output being regulated by parasympathetic activity mediated by muscarinic acetylcholine receptors (mAChRs) in the salivary gland cells responsible for saliva secretion (Baum, Ann NY Acad Sci. 1993;694:17-23; Cook et al., Physiology of the Gastrointestinal Tract. Raven, New York: 1994. pp. 1061-1117). In terms of mAChR subtype, both the M1 and M3 subtypes have been reported to be present in salivary glands, however, results have not been conclusive. While it is still under discussion which mAChR subtypes contribute to cholinergic control of salivation and to what extent, especially under physiological conditions, it is observed that, antagonists of the M3 muscarinic receptor often result in dry mouth as a side effect.

WO 07/027675 discloses pharmaceutical compositions comprising a therapeutically effective amount of a first compound and a therapeutically effective amount of a second compound. The first compound is an antimuscarinic or an anticholinergic agent and the second compound causes stimulation of salivary glands. The second compound of WO 07/027675 is a cholinergic agonist inter alia selected from the group consisting of pilocarpine, cevimeline, and amifostine or a pharmaceutically acceptable salt or prodrug thereof. The combination is based on the concept that at the receptor level, the adverse effect of the M2/M3 muscarinic antagonists is counteracted or negated with cholinergic agents that work in the opposite direction, but in concert with the intended therapy. In a case study, WO 07/027675 describes the combined use of oxybutynin and pilocarpine, whereby a combination of 5 mg oxybutynin and 5 mg pilocarpine results in a slightly increased saliva production compared to baseline.

Pilocarpine shows dose-dependent effects on salivation. In human trials, it was observed that 2.5, 5 and 10mg pilocarpine increased salivation (see Example 1 below). Yet, at clinically relevant doses (5mg and 10mg qd), a significant number of the patients suffered from sweating. Pilocarpine therefore alleviates the dry mouth symptoms, but it leads to hyperhidrosis as a further side effect.

Accordingly, there is further need for medication that alleviates dry mouth without causing further side effects such as hyperhidrosis.

The present inventors have found that this need can be met with certain positive allosteric modulators (PAM) of the muscarinic receptor. A PAM is an agent that binds to a site different from the ligand-binding site of the target and thereby positively alters the binding and function of the ligand. The finding underlying the present invention is surprising in at least two respects. First, the PAMs according to the present invention were found to be useful for use in the treatment of dry mouth, whereby some PAMs show this effect even in the absence of an anticholinergic agonist. Second, it was found that the compounds for use according to the present invention are useful for the treatment of dry mouth without causing sweating.

The compounds for use in the present invention and methods of making the same are known from EP-A 3 153 511 and EP-A 2 963 036. EP-A 3 153 511 describes the compounds for use according to the present invention as muscarinic M3 receptor positive allosteric modulators and their use for treating bladder and urinary tract diseases associated with bladder contractions via a muscarinic M3 receptor. The same is true of EP-A 2 963 036. Neither EP-A-3 153 511 nor EP-A 2 963 036 discloses or suggests that the compounds disclosed therein may be useful in the treatment of dry mouth.

The compounds for use according to the present invention are compounds with formula (I): 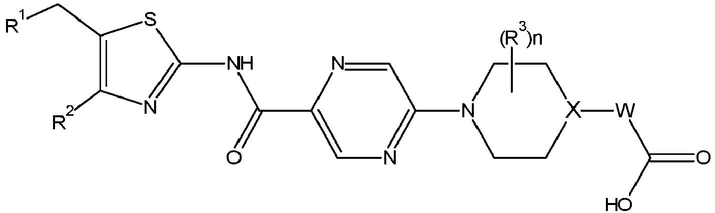 wherein:
R¹ is -N(-R¹¹)(-R¹²), or cyclic amino which may be substituted with 1 to 5 substituents selected from the group consisting of Group G and oxo,
R¹¹ is C1-6 alkyl,
R¹² is C1-6 alkyl which may be substituted with 1 to 5 substituents selected from substituents (b) to (o) of the Group G, or C3-8 cycloalkyl which may be substituted with 1 to 5 substituents selected from the Group G,
R² is aryl, a monocyclic aromatic hetero ring, or a bicyclic aromatic hetero ring, which each may be substituted with 1 to 5 substituents selected from the Group G,
R^{3'}s are the same as or different from each other, and are each C1-6 alkyl,
X is N or CH,
W is a bond or C1-6 alkylene, and
n is 0 or an integer of 1 to 4, wherein the
Group G is the group consisting of:
   (a) C1-6 alkyl which may be substituted with at least one group selected from the group consisting of -OH, -O-(C1-6 alkyl), -CN, -SO2-(C1-6 alkyl), and halogen,
   (b) -OH,
   (c) -O-(C1-6 alkyl) which may be substituted with at least one group selected from the group consisting of -OH, -O-(C1-6 alkyl), -CN, -SO2-(C1-6 alkyl), and halogen,
   (d) C3-8 cycloalkyl,
   (e) -O-(C3-8 cycloalkyl),
   (f) halogen,
   (g) -CN,
   (h) -SO2-(C1-6 alkyl),
   (i) -CO2-(C1-6 alkyl) and -COOH,
   (j) -CO-N(C1-6 alkyl)2, -CO-NH(C1-6 alkyl), and -CONH2,
   (k) -CO-(C1-6 alkyl),
   (l) -SO2-N(C1-6 alkyl)2, -SO2-NH(C1-6 alkyl), and -SO2NH2,
   (m) -N(C1-6 alkyl)2, -NH(C1-6 alkyl), and -NH2,
   (n) saturated hetero ring, and
   (o) -O-saturated hetero ring,
or pharmaceutically acceptable salts or esters thereof.

The "alkyl" is linear alkyl and branched alkyl. Accordingly, the "C1-6 alkyl" is linear or branched alkyl having 1 to 6 carbon atoms, and specific examples thereof include methyl, ethyl, n-propyl, isopropyl, n-butyl, isobutyl, sec-butyl, tert-butyl, n-pentyl, or n-hexyl; in one embodiment, methyl, ethyl, n-propyl, isopropyl, n-butyl, isobutyl, sec-butyl, or tert-butyl, each of which is C1-4 alkyl; in one embodiment, a group selected from the group consisting of methyl, ethyl, isopropyl, and isobutyl; and in one embodiment, a group selected from the group consisting of methyl and ethyl.

The "alkylene" is linear alkylene or branched alkylene. Accordingly, the "C1-6 alkylene" is linear or branched alkylene having 1 to 6 carbon atoms, and examples thereof include methylene, ethylene, trimethylene, tetramethylene, pentamethylene, hexamethylene, propylene, methylmethylene, ethylethylene, 1,2-dimethylethylene, or 1,1,2,2-tetramethylethylene; in one embodiment, C1-3 alkylene; in one embodiment, methylene or ethylene; in one embodiment, methylene; and in another embodiment, ethylene.

The "halogeno-C1-6 alkyl" is C1-6 alkyl substituted with at least one halogen atom; in one embodiment, C1-6 alkyl substituted with 1 to 5 halogen atoms; in one embodiment, difluoromethyl or trifluoromethyl; and in one embodiment, trifluoromethyl.

The "cycloalkyl" is a saturated hydrocarbon cyclic group. Accordingly, the "C3-8 cycloalkyl" is a saturated hydrocarbon cyclic group having 3 to 8 ring members, and specific examples thereof include cyclopropyl, cyclobutyl, cyclopentyl, cyclohexyl, cycloheptyl, or cyclooctyl; in one embodiment, cyclopropyl, cyclobutyl, cyclopentyl, or cyclohexyl, each of which is C3-6 cycloalkyl; and in one embodiment, cyclopropyl.

The "aryl" is a C6-14 monocyclic to tricyclic aromatic hydrocarbon cyclic group and includes a partially hydrogenated cyclic group thereof, and specific examples thereof include phenyl, naphthyl, tetrahydronaphthyl, indanyl, or indenyl; and in one embodiment, phenyl.

The "monocyclic aromatic hetero ring" is a monocyclic aromatic hetero ring group having 5 to 7 ring members, which has 1 to 4 hetero atoms selected from the group consisting of a nitrogen atom, an oxygen atom, and a sulfur atom as a ring-constituting atom, and specific examples thereof include pyrrolyl, pyrazolyl, imidazolyl, triazolyl, furyl, thienyl, oxazolyl, oxadiazolyl, thiazolyl, thiadiazolyl, pyridyl, pyridazinyl, pyrimidinyl, pyrazinyl, or azepanyl; in one embodiment, thienyl or pyridyl; and in one embodiment, thienyl.

The "bicyclic aromatic hetero ring" is a bicyclic aromatic hetero ring group in which the monocyclic aromatic hetero ring is fused with a benzene ring or monocyclic aromatic hetero ring and includes a partially hydrogenated ring group thereof, and specific examples thereof include indolyl, isoindolyl, indazolyl, benzotriazolyl, benzofuranyl, benzothienyl, benzooxazolyl, benzothiazolyl, quinolyl, isoquinolyl, cinnolinyl, quinazolinyl, quinoxalinyl, naphthyridinyl, furopyridyl, thienopyridyl, indolinyl, dihydrobenzofuranyl, dihydrobenzothienyl, dihydroquinolyl, tetrahydroquinolyl, dihydroisoquinolyl, tetrahydroisoquinolyl, dihydrofuropyridyl, or dihydrothienopyridyl; and in one embodiment, benzothienyl.

The "saturated hetero ring" is a 3- to 8-membered saturated ring group, which has 1 to 4 hetero atoms selected from the group consisting of a nitrogen atom, an oxygen atom, and a sulfur atom as a ring-constituting atom, and may be bridged with C1-6 alkylene, in which a sulfur atom as the ring-constituting atom may be oxidized. Specific examples thereof include azepanyl, diazepanyl, oxazepanyl, thiazepanyl, aziridinyl, azetidinyl, pyrrolidinyl, imidazolidinyl, piperidinyl, pyrazolidinyl, piperazinyl, azocanyl, thiomorpholinyl, thiazolindinyl, isothiazolindinyl, oxazolindinyl, morpholinyl, thiomorpholinyl, tetrahydrothiopyranyl, oxathioranyl, oxiranyl, oxetanyl, dioxiranyl, tetrahydrofuranyl, tetrahydropyranyl, and 1,4-dioxanyl.

The "cyclic amino" is a 4- to 7-membered group having a bond at a ring-constituting nitrogen atom in the saturated hetero ring. Specific examples thereof include aziridin-1-yl, azetidin-1-yl, pyrrolidin-1-yl, piperidin-1-yl, azepan1-yl, azocan-1-yl, morpholin-4-yl, thiomorpholin-4-yl, piperazin-1-yl, 1,4-diazepan-1-yl, 1,4-oxazepan-4-yl, or 1,4-thiazepan-4-yl; in one embodiment, pyrrolidin-1-yl, piperidin-1-yl, azetidin-1-yl, morpholin-4-yl, or piperazin-1-yl; and in one embodiment, pyrrolidin-1-yl or piperidin-1-yl.

The "halogen" means fluoro, chloro, bromo, or iodo; in one embodiment, fluoro, chloro, or bromo; in one embodiment, fluoro or chloro; in one embodiment, fluoro; and in another embodiment, chloro.

In the present specification, the expression "which may be substituted" means "which is not substituted" or "which is substituted with 1 to 5 substituents". Further, if it has a plurality of substituents, the substituents may be the same as or different from each other.

The acceptable substituent in the "cyclic amino which may be substituted", the "C3-8 cycloalkyl which may be substituted", the "aryl which may be substituted", the "monocyclic aromatic hetero ring which may be substituted", and the "bicyclic aromatic hetero ring which may be substituted" include substituents in Group G.

In preferred compounds R¹, R², W and n either alone or in combination of one or more of R¹, R², W and n, have the following meaning: R¹ is cyclic amino, preferably pyrrolidinyl, which may be substituted with 1 to 5 substituents selected from the group consisting of Group G and oxo; R² is aryl such as phenyl, a monocyclic aromatic hetero ring such as thienyl, or a bicyclic aromatic hetero ring, which each may be substituted with 1 to 5 substituents selected from the Group G, R³'s are the same as or different from each other, and are each hydrogen or C1-6 alkyl, especially methyl, W is a bond or C2 alkylene, and n is 0 or an integer of 1 to 4, and Group G is as defined above.

The compound of the formula (I) or a salt or ester thereof, in which R¹ is cyclic amino which may be substituted with 1 to 5 substituents selected from the group consisting of Group G and oxo is preferred. The compound of the formula (I) or a salt or ester thereof, in which R¹ is pyrrolidin-1-yl or piperidin-1-yl each substituted with 1 to 2 substituents selected from the group consisting of C1-6 alkyl and halogeno-C1-6 alkyl is also preferred. 2-methyl or 2-ethyl substituents such as 2-methyl-pyrrolidin-1-yl or 2-ethyl-pyrrolidin-1-yl are especially preferred.

A highly preferred compound according to the present invention is the compound of example 8 of EP-A 2 963 036 (hereinafter compound 1) and its pharmaceutical salts and esters:

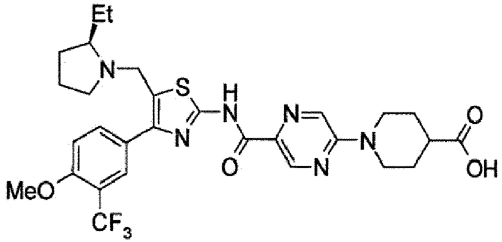

Compounds of formula (I) wherein X is N are especially preferred.

Even more preferred is compound 2 (see Example 144 in EP-B 3 153 511) in its free form or its esters or pharmaceutical salts thereof such as the di-maleate:

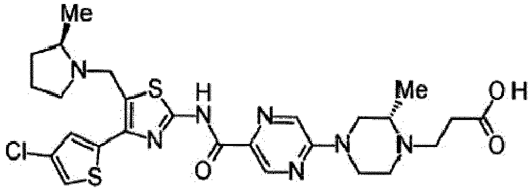

With regard to the compound of the formula (I), tautomers or geometrical isomers thereof may exist, depending on the kinds of the substituents. In the present specification, the compound of the formula (I) may be described in only one form of isomers in some cases, isolated forms of the isomers, or a mixture thereof.

Furthermore, some of the compounds of the formula (I) may have asymmetric carbon atoms or asymmetries in some cases, and correspondingly, the optical isomers thereof can exist. The present invention includes the isolated form of the optical isomer of the compound of the formula (I) or a mixture thereof.

In addition, a pharmaceutically acceptable prodrug of the compound represented by the formula (I) is also described. The pharmaceutically acceptable prodrug refers to a compound having a group which can be converted into an amino group, a hydroxyl group, a carboxyl group, or the like, by solvolysis or under a physiological condition. Examples of the groups forming the prodrug include those as described in Prog. Med., 5, 2157-2161 (1985) or "Pharmaceutical Research and Development" (Hirokawa Publishing Company, 1990), vol. 7, Drug Design, 163-198.

Moreover, the salt of the compound of the formula (I) is a pharmaceutically acceptable salt of the compound of the formula (I), and the compounds of the formula (I) may form an acid addition salt or a salt with a base, depending on the kinds of the substituents in some cases. Specifically, examples thereof include acid addition salts with inorganic acids such as hydrochloric acid, hydrobromic acid, hydroiodic acid, sulfuric acid, nitric acid, and phosphoric acid, and with organic acids such as formic acid, acetic acid, propanoic acid, oxalic acid, malonic acid, succinic acid, fumaric acid, maleic acid, lactic acid, malic acid, mandelic acid, tartaric acid, dibenzoyl tartaric acid, ditolyl tartaric acid, citric acid, methanesulfonic acid, ethanesulfonic acid, benzenesulfonic acid, p-toluenesulfonic acid, aspartic acid, and glutamic acid, and salts with metal anions such as sodium, potassium, magnesium, calcium, and aluminum, and with organic bases such as methylamine, ethylamine, and ethanolamine, salts with various amino acids such as acetyl leucine, lysine, and ornithine, or derivatives of amino acids, ammonium salts, and others.

In addition, the present invention also includes various hydrates or solvates, and crystal polymorph substances of the compound of the formula (I) and a salt thereof. In addition, the present invention also includes the compounds labelled with various radioactive or non-radioactive isotopes.

A pharmaceutical composition including one or two or more kinds of the compound of the formula (I) as an active ingredient can be prepared using an excipient which is usually used in the art, that is, an excipient for a pharmaceutical preparation, a carrier for a pharmaceutical preparation, and the like, according to a method usually used.

Administration can be accomplished either by oral administration via tablets, pills, capsules, granules, powders, solutions, and the like, or parenteral administration via injections, such as intraarticular, intravenous, and intramuscular injections, suppositories, transdermal liquid preparations, ointments, transdermal patches, transmucosal liquid preparations, transmucosal patches, inhalers, and the like. Oral administration is preferred.

As a solid composition for oral administration, tablets, powders, granules, and the like are used. In such a solid composition, one kind or two or more kinds of the active ingredients are mixed with at least one inactive excipient. In a conventional method, the composition may contain inactive additives such as a lubricant, a disintegrating agent, a stabilizer, or a solubilization assisting agent. If necessary, tablets or pills may be coated with a sugar or with a film of a gastric or enteric coating substance. Tablets are the preferred form of administration.

The liquid composition for oral administration includes pharmaceutically acceptable emulsions, solutions, suspensions, syrups, elixirs, or the like, and includes generally used inert diluents, for example, purified water or ethanol. The liquid composition may also include auxiliary agents such as a solubilization assisting agent, a moistening agent, and a suspending agent, sweeteners, flavors, aromatics, and antiseptics, in addition to the inert diluent.

The injections for parenteral administration include sterile aqueous or non-aqueous solution preparations, suspensions, or emulsions. The aqueous solvent includes, for example, distilled water for injection and saline. Examples of the non-aqueous solvent include alcohols such as ethanol. Such a composition may further include a tonicity agent, an antiseptic, a moistening agent, an emulsifying agent, a dispersing agent, a stabilizing agent, or a solubilizing assisting agent. These are sterilized, for example, by filtration through a bacteria retaining filter, blending of a bactericide, or irradiation. In addition, these can also be used by preparing a sterile solid composition and dissolving or suspending it in sterile water or a sterile solvent for injection prior to its use.

Examples of the agent for external use include ointments, hard plasters, creams, jellies, cataplasms, sprays, and lotions. The agent further contains generally used ointment bases, lotion bases, aqueous or non-aqueous liquid preparations, suspensions, emulsions, or the like.

As transmucosal delivery is contemplated, e.g. by inhalation or transnasal delivery, the pharmaceutical composition can take the form of a solid, liquid, or semi-solid and it can be prepared in accordance with any method known in the art. For example, a known excipient, and also a pH adjusting agent, an antiseptic, a surfactant, a lubricant, a stabilizing agent, a thickening agent, or the like may be appropriately added thereto. For the administration, an appropriate device for inhalation or blowing can be used. For example, a compound may be administered alone or as a powder of formulated mixture, or as a solution or suspension in combination with a pharmaceutically acceptable carrier, using a known device or sprayer such as a metered administration inhalation device. A dry powder inhaler or the like may be for single or multiple administration use, and a dry powder or a powder-containing capsule may be used. Alternatively, this may be in a form such as a pressurized aerosol spray that uses an appropriate propellant agent, for example, a suitable gas such as chlorofluoroalkanes, and carbon dioxide, or other forms.

In the case of oral administration, the daily oral an transmucosal dose is usually from 0.001 mg/kg body weight to 100 mg/kg body weight, preferably from 0.01 mg/kg body weight to 30 mg/kg body weight, more preferably from 0.01 mg/kg body weight to 10 mg/kg body weight and most preferably 0.01 mg/kg body weight to 5 mg/kg body weight. For compound 1 a preferred daily dose is 0.1 to 5 mg/kg body weight or more preferably 0.1 to 2 or 3 mg/kg body weight. For compound 1 a preferred daily dose is 0.01 to 5 mg/kg body weight and more preferred 0.01 to 2.5 mg/kg body weight. The daily oral or transmucosal dose can be administered in one portion or divided into 2 to 4 portions. In the case of intravenous administration, the daily dose correspond to the above oral doses, but it is preferably lower by a factor of 10. The dose is appropriately decided in response to the individual case by taking the symptoms, the age, and the gender, and the like into consideration. For compound (1), daily doses of 30 to 300, preferably 30 to 250 and more preferably 30 to 150 or 30 to 90 mg are useful. For compound 2, daily doses of 100 mg or higher especially between 100 and 200 mg such as 150 mg are preferential.

Although there are differences depending on the route of administration, the dosage form, the administration site, and the type of the excipient or additive, the pharmaceutical composition of the present invention comprises 0.01% by weight to 100% by weight of, as an embodiment, 0.01% by weight to 50% by weight of, one or more of the compound of the formula (I) or a salt or ester thereof which is the active ingredient.

The compound of the formula (I) may be used in combination with various agents that cause dry mouth, such as anticholinergic agents. Examples for anticholinergic agents are atropine, aclidinium, benztropine, cyclopentolate, diphenhydramine, doxylamine, dimenhydrinate, diclomine, darifenacin, flavoxate, hydroxyzine, ipatropium, mebeverine, oxybutynin, pirenzepine, procyclidine, scopolamine, solifenacin, topicamide, tolterodine, tiotropium, trospium and their pharmaceutically acceptable salts.

The compound may also be administered in combination with parasympathomimetica such as pilocarpine. Parasympathomimetic drugs may be used to reduce dry mouth, yet they cause hyperhidrosis. A combination of such parasympathomimetic compounds with the compounds according to formula (I) allows to reduce the dose of the parasympathomimetic and thereby treat dry mouth while reducing or eliminating the unwanted sweating. Compound 2 can treat dry mouth without causing hyperhidrosis when used on its own.

The compounds of the invention and the other therapeutic agents may be administered simultaneously, or separately and continuously, or at a desired time interval. The preparations to be co-administered may be a blend or may be prepared individually.

### Example 1 (Reference)

A Phase 1 study to investigate the effect of pilocarpine on salivary secretion was conducted in 12 healthy subjects. The study was conducted as a randomized, subject- and investigator-blinded, placebo-controlled, oral dose clinical study using a 5-way balanced extra period design.

**Table 1 Dosing Plan for Each Treatment Sequence**

| | **Period 1** | **Period 2** | **Period 3** | **Period 4** | **Period 5** |
|---|---|---|---|---|---|
| **Sequence 1** | 2.5 mg | 5 mg | 10 mg | Placebo | Placebo |
| **Sequence 2** | 5 mg | Placebo | 2.5 mg | 10 mg | 10 mg |
| **Sequence 3** | 10 mg | 2.5 mg | Placebo | 5 mg | 5 mg |
| **Sequence 4** | Placebo | 10 mg | 5 mg | 2.5 mg | 2.5 mg |

Pilocarpine capsules containing 2.5 mg were administered orally (1 capsule for a 2.5 mg dose, 2 capsules for a dose of 5.0 mg pilocarpine and 4 capsules for a dose of 10 mg pilocarpine). Subjects were admitted to the clinical unit on day -1 and were residential for a period of 6 days and 5 nights. Subjects were randomized to 1 of 4 treatment sequences on day 1 prior to study drug administration. Each study period was approximately 1 day and subjects received the different oral doses of the study drug (pilocarpine or placebo to match) in periods 1 through 5. There was a 4-hour saliva collection period following every dose and a washout period of at least 24 hours between doses.

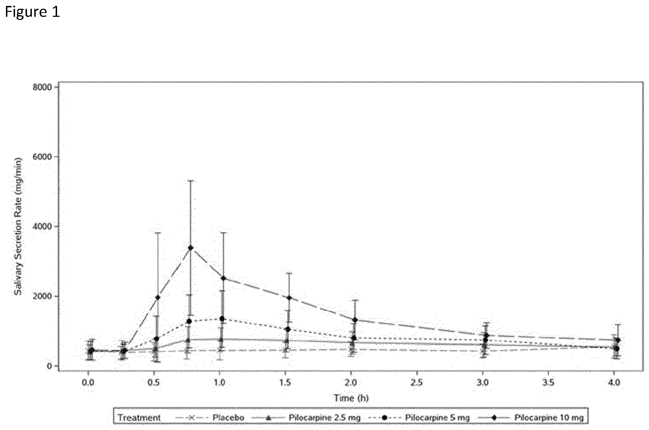

To assess the pharmacodynamic effect of pilocarpine on saliva secretion, saliva samples were collected over 5-minute intervals from pre-dose until 4 hours post-dose at the timepoints 0.5, 0.75, 1.0, 1.5, 2.0, 3.0 and 4.0h. The salivary secretion rate over time is shown in Figure 1.

The most commonly reported side effect for periods 1 to 4 was hyperhidrosis. At a pilocarpine dose of 5mg, this side effect was reported by one participant (8.3%). At a pilocarpine dose of 10mg, this side effect was reported by 50% of the participants.

### Example 2

The impact of compound 1 on salivation and its side effects were studied in a randomized, subject- and investigator-blinded, placebo-controlled, 3-sequence, escalating incomplete block crossover study with 12 participants in residence. Three single doses of compound 1 (a low [10 mg], medium [30 mg] and high [90 mg] dose) or matching placebo were given to one cohort consisting of 12 subjects. Dose levels of compound 1 were investigated in ascending order. The sequences are given below:

**Table 2**

| | **Period 1** | **Period 2** | **Period 3** |
|---|---|---|---|
| **Sequence 1** | Placebo | 30 mg Compound 1 | 90 mg Compound 1 |
| **Sequence 2** | 10 mg Compound 1 | placebo | 90 mg Compound 1 |
| **Sequence 3** | 10 mg Compound 1 | 30mg Compound 1 | placebo |

Each subject received single oral doses of 5 mg pilocarpine and 10, 30 or 90 mg of compound 1 or placebo on day 1 after an overnight fast (i.e., no caloric intake for at least 10 hours prior to study drug administration and for another 4.5 hours following study drug administration). Each dose was followed by a 6-hour saliva sampling period. Subjects were required to drink 150 mL of water every hour from 1 hour prior to pilocarpine dosing until 6 hours after pilocarpine dosing (i.e., 150 mL at 1 hour pre-dose; 240 mL at time of dosing [0 hours]; 75 mL at 1 hour, 1 hour 30 minutes post-dose (30 minutes post-dose water consumption covered by the 240 mL water consumed to swallow study drug); 150 mL at 2, 3, 4, 5, 6 hours post-dose). Water was allowed ad libitum after 6 hours post-dose.

Assessment of salivary secretion was performed in all subjects. During the assessment, the subject sat in an upright position. Immediately before the collection was started, the subjects swallowed all saliva and then inclined the head forward so that all newly produced saliva flowed out over the lips. The saliva was collected in a beaker over a period of 5 minutes for each time point. The start and the end time of the interval were recorded. The results are summarised in Table 3 below.

In the placebo and pilocarpine group, the LS mean of salivation rates was 786.7 mg/min. In the groups receiving compound 1 (at 10, 30, and 90 mg) and pilocarpine, the LS mean of salivation rates were higher, namely 859.0 to 892.8 mg/min. Similarly, the AUCₛₐₗ LS mean data for the placebo and pilocapine group was lower than that for the groups receiving compound 1 and pilocarpine. The same trend is also seen in the E_{max sal} LS mean data. Side effects were not reported when using compound 1 at any dosage.

As shown in example 1, the salivation induced by pilocarpine can be increased by increasing the pilocarpine dose. However, this increase comes at the expense that many patients suffer from hyperhidrosis. On the basis of the mean data shown below a combination of pilocarpine and compound 1 may allow for increased salivation without causing hyperhidrosis.

**Table 3**

| **Parameter** | **n** | **LS Mean** | **LS Mean Difference Treatment - Placebo** |
|---|---|---|---|
| **Salivary Secretion Rates - Overall Time Points (mg/min)** | | | |
| Placebo + Pilocarpine 5 mg | 12 | 786.7 | |
| Compound 110 mg + Pilocarpine 5 mg | 8 | 891.6 | 104.9 |
| Compound 1 30 mg + Pilocarpine 5 mg | 8 | 859.0 | 72.3 |
| Compound 1 90 mg + Pilocarpine 5 mg | 8 | 892.8 | 106.1 |

| **AUCₛₐₗ (h•mg/min)** | | | |
|---|---|---|---|
| Placebo + Pilocarpine 5 mg | 12 | 4202.7 | |
| Compound 110 mg + Pilocarpine 5 mg | 8 | 4616.1 | 413.3 |
| Compound 1 30 mg + Pilocarpine 5 mg | 8 | 4696.0 | 493.3 |
| Compound 1 90 mg + Pilocarpine 5 mg | 8 | 4749.1 | 546.3 |

| **E_{max, sal} (mg/min)** | | | |
|---|---|---|---|
| Placebo + Pilocarpine 5 mg | 12 | 1389.8 | |
| Compound 110 mg + Pilocarpine 5 mg | 8 | 1647.2 | 257.3 |
| Compound 1 30 mg + Pilocarpine 5 mg | 8 | 1391.8 | 2.0 |
| Compound 1 90 mg + Pilocarpine 5 mg | 8 | 1449.5 | 59.7 |

| | | | |
|---|---|---|---|
| LS: least squares. AUCₛₐₗ: Area under salivation concentration time curve, total amount of saliva E_{max sal}: Maximum pharmacodynamic effect for salivary secretion | | | |

### Example 3

In the first part of a randomized, subject- and investigator-blinded, placebo-controlled, single ascending oral dose, parallel group study comprising of 7 cohorts (cohorts 1.1 to 1.7) compound 2 was administered at dose levels of 1, 3, 10, 30, 100, and 150mg. Each cohort consisted of 8 healthy non-elderly male and female subjects who were randomized to receive compound 2 or matching placebo in a 3:1 ratio.

After a screening period of 21 days, eligible subjects who had signed an informed consent form (ICF) to participate in the study were residential for a single treatment period of 5 days/4 nights. Subjects were admitted to the clinical unit on day -1. After randomization on day 1, subjects received a single oral dose of compound 2 or matching placebo under fasting. Each cohort was completed with an end-of-study visit, which took place 5 to 9 days after the last pharmacokinetic sample had been collected (or early discontinuation from the study).

The second part of the study investigated, multiple ascending oral doses in 4 cohorts (cohorts 2.1 to 2.4). Each cohort consisted of 12 healthy non-elderly male and female subjects, one optional cohort (cohort 2.5) consisting of 12 healthy nonelderly male and female subjects and one cohort (cohort 2.6) consisting of 12 healthy elderly male and female subjects who were randomized to receive compound 2 or matching placebo in a 3:1 ratio.

Actual dose levels were 10, 30, 100 and 150 mg of compound 2 (cohorts 2.1 to 2.4, respectively). The optional cohort 2.5 was not enrolled. The actual dose level for the elderly cohort (cohort 2.6) was 150 mg compound 2.

Again, sentinel dosing was employed for all cohorts. The first 2 subjects within a cohort (1 subject received compound 2 and 1 subject received matching placebo) were dosed at least 5 minutes apart. These 2 subjects were monitored for safety for 48 hours after receiving the first dose while dosing was continued. The remaining 10 subjects (8 with compound 2 and 2 with matching placebo) were divided into 2 sub-cohorts and were dosed at least 48 hours after the first 2 subjects with 48 hours between the 2 sub-cohorts.

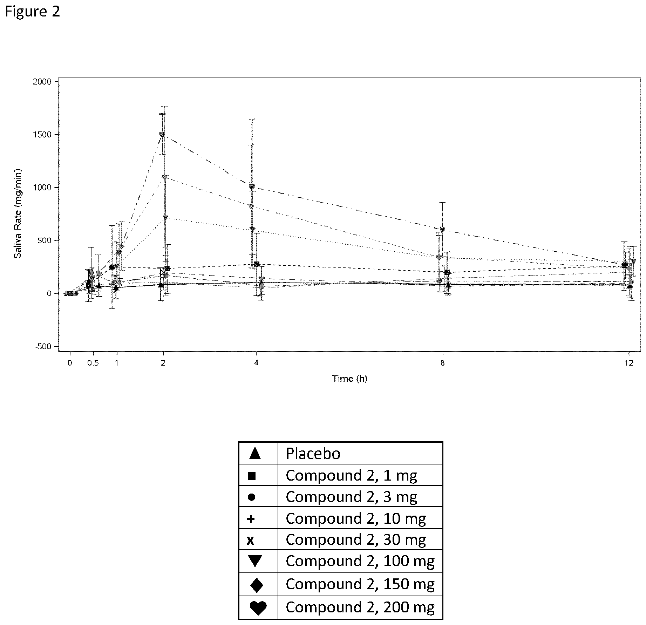

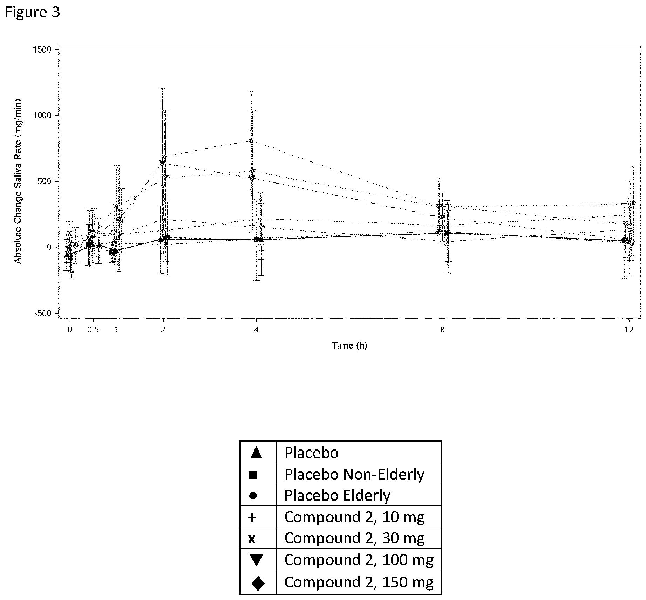

In both studies compound 2 increased salivary secretion rates, while hyperhidrosis was not reported as a side effect. The results of the first study (single dose) are shown in Figure 2 and the results of the second study (multiple dose) are shown in Figure 3.

The above data indicates that the compounds of the present invention are beneficial in that they increase salivation either alone or in combination with e.g. pilocarpine. Moreover, clinical studies do not suggest that the compounds of the invention cause hyperhidrosis. Hence the compounds of the invention seem suited to remove the most serious side effect of the current dry mouth treatments.

## Claims

1. Compound with formula (I) 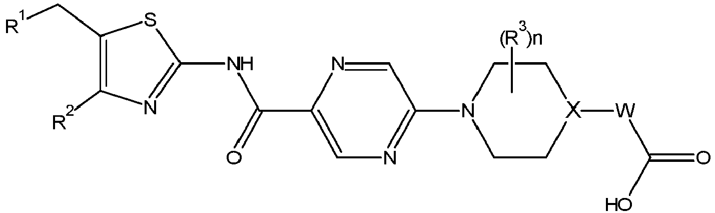
R¹ is -N(-R¹¹)(-R¹²), or cyclic amino which may be substituted with 1 to 5 substituents selected from the group consisting of Group G and oxo,
R¹¹ is C1-6 alkyl,
R¹² is C1-6 alkyl which may be substituted with 1 to 5 substituents selected from substituents (b) to (o) of the Group G, or C3-8 cycloalkyl which may be substituted with 1 to 5 substituents selected from the Group G,
R² is aryl, a monocyclic aromatic hetero ring, or a bicyclic aromatic hetero ring, which each may be substituted with 1 to 5 substituents selected from the Group G,
R³'s are the same as or different from each other, and are each C1-6 alkyl,
X is N or CH,
W is a bond or C1-6 alkylene, and
n is 0 or an integer of 1 to 4, wherein the
Group G is the group consisting of:
(a) C1-6 alkyl which may be substituted with at least one group selected from the group consisting of -OH, -O-(C1-6 alkyl), -CN, -SO2-(C1-6 alkyl), and halogen,
(b) -OH,
(c) -O-(C1-6 alkyl) which may be substituted with at least one group selected from the group consisting of -OH, -O-(C1-6 alkyl), -CN, -SO2-(C1-6 alkyl), and halogen),
(d) C3-8 cycloalkyl,
(e) -O-(C3-8 cycloalkyl),
(f) halogen,
(g) -CN,
(h) -SO2-(C1-6 alkyl),
(i) -CO2-(C1-6 alkyl) and -COOH,
(j) -CO-N(C1-6 alkyl)2, -CO-NH(C1-6 alkyl), and -CONH2,
(k) -CO-(C1-6 alkyl),
(l) -SO2-N(C1-6 alkyl)2, -SO2-NH(C1-6 alkyl), and -SO2NH2,
(m) -N(C1-6 alkyl)2, -NH(C1-6 alkyl), and -NH2,
(n) saturated hetero ring, and
(o) -O-saturated hetero ring,
or pharmaceutically acceptable salts or esters thereof
for use in the treatment of dry mouth.

2. The compound for use according to claim 1, wherein R¹ is cyclic amino which may be substituted with 1 to 5 substituents selected from the group consisting of Group G.

3. The compound for use according to claim 1 or claim 2, wherein R¹ is pyrrolidin-1-yl or piperidin-1-yl, optionally substituted with 1 to 2 substituents selected from the group consisting of C1-6 alkyl and halogeno-C1-6 alkyl

4. The compound for use according to the preceding claims, which is 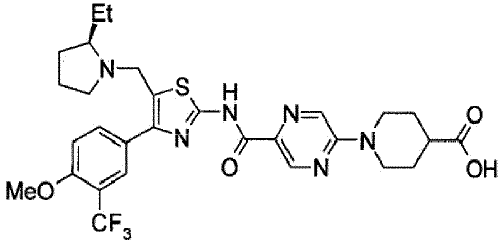 or

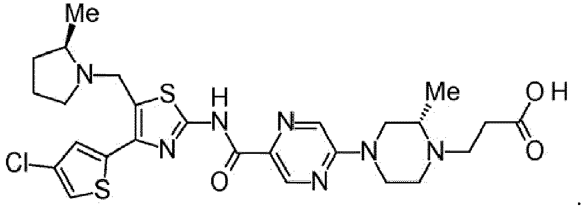

5. The compound for use according to any one of the preceding claims, wherein the dry mouth is associated with Sjörgren's disease, radiation therapy or anticholinergic antagonists.

6. The compound for use according to any one of the preceding claims, wherein the compound with formula (I) is co-administered with an anticholinergic antagonist.

7. The compound for use according to claim 6, wherein the anticholinergic antagonist is selected from atropine, aclidinium, benztropine, cyclopentolate, diphenhydramine, doxylamine, dimenhydrinate, diclomine, darifenacin, flavoxate, hydroxyzine, ipatropium, mebeverine, oxybutynin, pilocarpine, pirenzepine, procyclidine, scopolamine, solifenacin, topicamide, tolterodine, tiotropium, trospium and their pharmaceutically acceptable salts.

8. A pharmaceutical composition comprising an anticholinergic antagonist and a positive allosteric modulator as defined any one of the preceding claims.

9. A pharmaceutical composition according to claim 8, comprising pilocarpine.
